# DEMANDE DE BREVET EUROPEEN

(11) **EP 2 072 144 A1**
(43) Date de publication de la demande: **24.06.2009**
(21) Numéro de dépôt: 08021589.0
(22) Date de dépôt: 12.12.2008
(51) Int. Cl.: B05B 12/02, B05B 12/10, B05B 12/12, B05B 17/06, A61L 9/12

(54) **Procédé de commande d'un appareil de nébulisation de liquides dans l'air**

(30) Priorité: 17.12.2007 FR 0708773
(71) Demandeur: Osmooze, 26270 Loriol sur Drome (FR)
(72) Inventeur: Millet, Jean-Claude, 26800 Etoile sur Rhone (FR)
(74) Mandataire: Marchand, André

(57) **Abrégé**

L'invention concerne un procédé de commande d'un ou plusieurs appareils de nébulisation de liquides dans l'air (DF1), le procédé comprenant des étapes de commande de l'appareil pour nébuliser un liquide dans l'air conformément à des cycles de nébulisation durant lesquels un liquide est nébulisé dans l'air, les cycles de nébulisation étant espacés par des périodes de repos, et d'ajustement de la durée des périodes de repos en fonction d'une quantité moyenne de liquide à nébuliser par unité de temps sélectionnée, et en fonction d'un paramètre de concentration en produit actif du liquide à nébuliser. Le procédé permet avec un même appareil de traiter des volumes dans un rapport 100, typiquement de 7 à 700 m³.

## Description

La présente invention concerne les appareils de nébulisation de liquides dans l'air, à des fins d'humidification ou de rafraîchissement de l'air, ou pour diffuser notamment des produits assainissants, désodorisants, désinfectants, ou des parfums.

Certains appareils de nébulisation de liquide comportent un logement pour recevoir une bombe aérosol d'un liquide à diffuser, et un mécanisme pour déclencher périodiquement l'émission du produit sous forme d'aérosol pendant une durée fixe. Pour émettre du liquide sous forme d'aérosol, le mécanisme comprend un actionneur pour ouvrir une valve en appuyant sur la buse de la bombe. Des appareils de ce type sont commercialisés sous les marques Microburst 3000® et Microburst 9000Ⓡ. Ces appareils ne peuvent traiter que des volumes dans une gamme limitée, de 40 m3 à 170 m3 environ, et présentent une autonomie également relativement limitée selon la taille de la bombe aérosol qui peut atteindre 6 mois pour les plus petits volumes et 1,5 mois pour les plus grands volumes.

Un autre type d'appareil de nébulisation de liquide est par exemple décrit dans la demande de brevet FR 2 877 241. Cet appareil comprend un tube capillaire présentant une extrémité formant une buse d'éjection d'un liquide, un réservoir d'alimentation relié au tube capillaire par une canalisation, un moyen vibrant pour entraîner le tube capillaire en vibration afin qu'il éjecte des gouttelettes de liquide en un jet de nébulisation, et des moyens d'excitation pour appliquer au moyen vibrant un signal d'excitation.

La demande de brevet WO 00/78467 prévoit de programmer un appareil de nébulisation de liquides dans l'air de manière à produire chez les usagers une perception de "pic d'odeur". Une telle perception est obtenue lorsque le parfum est diffusé périodiquement en une certaine quantité durant des cycles nébulisation séparés par des périodes de repos. La durée des périodes de repos est calculée en prenant en compte les caractéristiques neurosensorielles olfactives des individus. Il peut en outre être prévu un temps d'attente de nature à laisser se développer le désir de l'usager, afin de renforcer son plaisir au moment de l'arrivée du pic d'odeur.

Ce mode de nébulisation en pic d'odeur s'avère optimum en terme de rendement (perception olfactive globale par rapport à la quantité de parfum diffusée) sur une période de plusieurs minutes à quelques heures. En revanche, une nébulisation continue présente un mauvais rendement. En effet, en raison des caractéristiques neurosensorielles humaines et en particulier du phénomène d'accoutumance, la perception d'une odeur s'atténue et disparaît au-delà de quelque minutes, sauf si l'intensité de l'odeur est excessive et donc induit un écoeurement pouvant être insupportable.

Pour éviter ce phénomène d'accoutumance, certains appareils sont programmés pour changer de temps en temps la nature du parfum qui est diffusé. Il existe également des appareils pilotés par un ordinateur selon un programme par exemple en association avec des données multimédia. D'autres appareils sont programmés pour produire une odeur d'accueil dans un lieu occupé de manière temporaire. A cet effet, ils sont associés à une détection de présence pour que l'odeur d'accueil soit émise lorsqu'une personne est détectée.

D'une manière générale, les appareils précités ne permettent pas de produire le meilleur rendement olfactif pour toutes les combinaisons possibles de paramètres liés au rendement olfactif, et notamment l'ambiance à créer sur le plan olfactif (accueil, ambiance permanente, pic d'odeur), et aux caractéristiques du lieu à traiter (volume, fonction). Ils ne permettent pas plus de programmer à volonté les cycles de diffusion en fonction d'autres critères, y compris en admettant de s'éloigner de rendements olfactifs optimaux, notamment si les produits diffusés ne sont pas des parfums.

Par ailleurs, les appareils précités sont adaptés pour traiter une gamme limitée de volumes. En effet, pour traiter de plus petits volumes, la durée des périodes de repos entre les cycles de nébulisation de parfum peut être augmentée, mais cela risque d'affecter la stabilité de la perception olfactive, notamment pour émettre une odeur d'accueil dans un lieu fréquenté d'une manière temporaire. La durée des cycles de nébulisation de parfum et donc la quantité de parfum diffusée peut être diminuée, mais cette possibilité présente des limites. La sensation olfactive d'un parfum dépend de sa concentration dans l'air ambiant. En outre, le rendu olfactif n'est pas directement proportionnel à la quantité de parfum présente dans l'air. En dessous d'un certain seuil, il n'y a pas de perception. Au-delà de ce seuil, la perception est croissante d'une manière non linéaire et variable d'un parfum à l'autre. Par ailleurs, lorsque la durée des cycles de nébulisation est inférieure à une certaine valeur de l'ordre de 50 ms, il est difficile de contrôler le débit du liquide ainsi diffusé.

A l'inverse, pour traiter de plus grands volumes, la durée des périodes de repos peut être diminuée, mais cela affecte l'autonomie de l'appareil en ce qui concerne son alimentation en liquide à diffuser et son alimentation électrique si celui-ci est alimenté par une source d'énergie limitée, par exemple par des piles électriques. La durée de chaque cycle de nébulisation peut également être augmentée, ce qui affecte également la consommation de l'appareil en liquide à diffuser et la consommation électrique de ce dernier. En outre, pour des raisons fonctionnelles (notamment dissipations thermiques dans le ou les actionneurs qui peuvent introduire des dérives de fonctionnement), il est préférable de ne pas augmenter la durée des cycles de nébulisation de liquide au-delà de quelques secondes. Par ailleurs, il peut être constaté que si la quantité de liquide nébulisé excède environ 12 g par jour pour un appareil, des gouttes du liquide nébulisé peuvent se déposer au voisinage de l'appareil. Ce phénomène se produit avec des liquides ayant une faible teneur en solvants légers ou volatils, c'est-à-dire dont le point éclair est inférieur à 62°C pour satisfaire à des contraintes environnementales et de sécurité.

En définitive, aucun des appareils précités ne permet de satisfaire aux besoins réels des usagers, et de s'adapter au lieu où l'appareil est utilisé. En effet, leur mode de fonctionnement ne peut pas réellement s'adapter aux goûts et à la sensibilité olfactive de tous les usagers. Leur mode de fonctionnement ne peut pas non plus s'adapter notamment à la taille et à la fonction de la pièce, ni aux conditions de ventilation, et au lieu d'implantation de l'appareil dans la pièce. En particulier, les appareils précités ne permettent pas de traiter indifféremment des volumes inférieurs à 20 m3 et supérieurs à 300 m3. En outre, les appareils qui peuvent traiter jusqu'à 300 m3 ne peuvent pas être adaptés à des volumes inférieurs à 75 m3, et les appareils adaptés aux volumes jusqu'à 30 m3 ne peuvent traiter des volumes excédant les 120 m3.

Il est donc souhaitable de prévoir un appareil de nébulisation de parfum qui soit polyvalent afin de pouvoir créer une odeur d'ambiance ou des pics d'odeurs dans des pièces de 15 à 360 m3, ou une bulle olfactive de 1 à 10 m de diamètre.

Il est également souhaitable de parvenir à ce résultat à partir d'un unique appareil moyennant des modifications les plus limitées possibles.

Dans le cadre d'un usage professionnel, il est également souhaitable que l'appareil présente une autonomie électrique et en liquide à nébuliser aussi grande que possible, pour des raisons d'économie et afin de limiter les opérations de maintenance de l'appareil. Pour cette dernière raison, il est également souhaitable que l'autonomie électrique et l'autonomie en liquide à nébuliser soient en phase, et en particulier, que la durée de vie des piles soit un multiple de celle des cartouches de liquide.

Dans un mode de réalisation, il est prévu un procédé de commande d'un appareil de nébulisation de liquides dans l'air, le procédé comprenant des étapes de : commande de l'appareil pour nébuliser un liquide dans l'air conformément à des cycles de nébulisation durant lesquels un liquide est nébulisé dans l'air, les cycles de nébulisation étant espacés par des périodes de repos, et ajustement de la durée des périodes de repos en fonction d'une quantité moyenne de liquide à nébuliser par unité de temps sélectionnée. Selon un mode de réalisation, le procédé comprend une étape d'ajustement de la durée des périodes de repos en fonction d'un paramètre de concentration en produit actif du liquide à nébuliser.

Selon un mode de réalisation, le procédé comprend une étape d'ajustement de la durée des cycles de nébulisation ou de la quantité de liquide à nébuliser à chaque cycle de nébulisation en fonction de la quantité de moyenne de liquide à nébuliser par unité de temps sélectionnée et du paramètre de concentration en produit actif contenu dans le liquide à nébuliser.

Selon un mode de réalisation, la durée de chaque cycle de nébulisation est ajustée à une valeur comprise entre une centaine de millisecondes et quelques secondes, et la durée de chaque période de repos entre les cycles de nébulisation est ajustée à une valeur comprise entre plusieurs dizaines de secondes et quelques minutes.

Selon un mode de réalisation, l'ajustement de la durée des périodes de repos en fonction de la concentration en produit actif du liquide contenu dans la cartouche peut être effectué lorsque la concentration en produit actif présente une valeur comprise entre 2,5% et 15%, et de préférence entre 2,5% et 60%.

Selon un mode de réalisation, le procédé comprend une étape d'ajustement de la durée des cycles de nébulisation en fonction de la température ambiante fournie par un capteur de température.

Selon un mode de réalisation, le procédé comprend une étape d'ajustement de la durée des périodes de repos et/ou des cycles de nébulisation en fonction d'une information fournie par un capteur.

Selon un mode de réalisation, les durées respectives des périodes de repos successives sont ajustées de manière à produire des "pics d'odeurs", en tenant compte de caractéristiques neurosensorielles olfactives humaines et en prévoyant un temps supplémentaire d'attente.

Selon un mode de réalisation, la concentration en produit actif du liquide à nébuliser est sélectionnée en fonction du volume à traiter par l'appareil et afin que la durée de fonctionnement de piles électriques d'alimentation de l'appareil soit un multiple entier au moins égal à 1 de la durée d'une cartouche amovible alimentant l'appareil en liquide à nébuliser.

Selon un mode de réalisation, le procédé comprend une étape de configuration du mode de fonctionnement de l'appareil consistant à déterminer la durée des périodes de repos et des cycles de nébulisation ou la quantité de liquide à nébuliser à chaque cycle de nébulisation en fonction d'au moins un paramètre appartenant à l'ensemble comprenant des dimensions et une fonction d'une pièce où est installé l'appareil, un nombre d'appareils installés dans la pièce, la position de l'appareil dans la pièce par rapport à des ouvertures et des bouches d'aération, un mode de diffusion du liquide à nébuliser, la consommation électrique de l'appareil, et la consommation de l'appareil en liquide à nébuliser.

Selon un mode de réalisation, le procédé comprend la transmission de paramètres de fonctionnement à une pluralité d'appareils de nébulisation de liquides dans l'air.

Dans un autre mode de réalisation il est prévu un appareil de nébulisation de liquides dans l'air, comprenant : un circuit de nébulisation, une cartouche amovible contenant un liquide à nébuliser relié au circuit de nébulisation, et une unité de commande du circuit de nébulisation configurée pour nébuliser le liquide dans l'air conformément à des cycles de nébulisation durant lesquels un liquide est nébulisé dans l'air, les cycles de nébulisation étant espacés par des périodes de repos, l'unité de commande étant configurée pour ajuster la durée des périodes de repos en fonction d'une quantité moyenne de liquide à nébuliser par unité de temps sélectionnée. Selon un mode de réalisation, l'unité de commande est configurée pour ajuster la durée des périodes de repos en fonction d'un paramètre de concentration en produit actif du liquide à nébuliser.

Selon un mode de réalisation, l'unité de commande est configurée pour ajuster la durée des cycles de nébulisation en fonction de la quantité moyenne de liquide à nébuliser sélectionnée et du paramètre de concentration en produit actif contenu dans le liquide à nébuliser.

Selon un mode de réalisation, l'unité de commande est configurée pour ajuster la durée de chaque cycle de nébulisation à une valeur comprise entre une centaine de millisecondes et quelques secondes, et ajuster la durée de chaque période de repos entre les cycles de nébulisation à une valeur comprise entre plusieurs dizaines de secondes et quelques minutes.

Selon un mode de réalisation, l'unité de commande est configurée pour pouvoir ajuster la durée des périodes de repos en fonction de la concentration en produit actif du liquide contenu dans la cartouche, lorsque la concentration en produit actif présente une valeur comprise entre 2,5% et 15%, et de préférence entre 2,5% et 60%.

Selon un mode de réalisation, l'unité de commande est configurée pour ajuster la durée des cycles de nébulisation en fonction de la température ambiante fournie par un capteur de température relié à l'unité de commande .

Selon un mode de réalisation, l'unité de commande est configurée pour ajuster la durée des périodes de repos et/ou des cycles de nébulisation en fonction d'une information fournie par un capteur relié à l'unité de commande .

Selon un mode de réalisation, l'unité de commande est configurée pour déterminer les durées respectives de périodes de repos successives de manière à produire des "pics d'odeurs", en tenant compte de caractéristiques neurosensorielles olfactives humaines et en prévoyant un temps supplémentaire d'attente.

Selon un mode de réalisation, l'appareil comprend un dispositif de transmission connecté à l'unité de commande, pour recevoir d'un dispositif de commande à distance des paramètres de fonctionnement, l'unité de commande étant configurée pour déterminer la durée des périodes de repos et/ou la durée des cycles de nébulisation en fonction des paramètres reçus.

Selon un mode de réalisation, les paramètres reçus du dispositif de commande par l'unité de commande comprennent des dimensions et une fonction d'une pièce où est installé l'appareil, ainsi qu'un mode de diffusion du liquide à nébuliser.

Selon un mode de réalisation, l'unité de commande est configurée pour transmettre vers le dispositif de commande à distance des informations d'état de l'appareil.

Selon un mode de réalisation, le dispositif de transmission est relié au dispositif de commande à distance par l'intermédiaire d'une liaison sans fil ou filaire.

Selon un mode de réalisation, l'appareil est configuré pour retransmettre un message provenant du dispositif de commande à distance et destiné à un autre appareil, et retransmettre un message reçu d'un autre appareil et destiné au dispositif de commande à distance.

Dans un autre mode de réalisation, il est prévu un système de nébulisation de liquides dans l'air, comprenant plusieurs appareils de nébulisation de liquides dans l'air. Selon un mode de réalisation, les appareils sont conformes à celui tel que défini ci-avant.

Selon un mode de réalisation, chacun des appareils est configuré pour retransmettre un message provenant du dispositif de commande à distance et destiné à un autre des appareils, et retransmettre un message reçu d'un autre des appareils et destiné au dispositif de commande à distance.

Des modes de réalisation de l'invention seront décrits dans ce qui suit, à titre non limitatif en relation avec les figures jointes parmi lesquelles :
- la figure 1 représente un appareil de nébulisation de liquides du type à tube capillaire vibrant,
- la figure 2 est une vue schématique d'un premier mode de réalisation d'un appareil de nébulisation,
- la figure 3 est une vue schématique d'un second mode de réalisation d'un appareil de nébulisation,
- la figure 4 est une vue schématique d'une installation comportant plusieurs appareils de nébulisation.

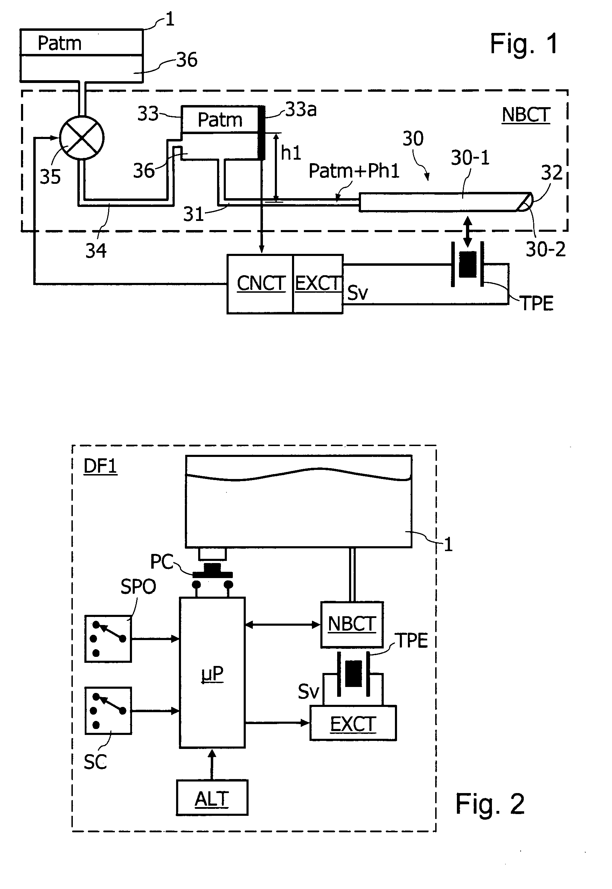

La figure 1 représente un schéma de principe d'un appareil de nébulisation du type à tube capillaire vibrant. L'appareil comprend un réservoir principal 1 contenant un liquide 36 à nébuliser, et un circuit de nébulisation NBCT alimenté par le réservoir 1. Le circuit de nébulisation NBCT comprend une tête de nébulisation 30, un réservoir intermédiaire 33 contenant également du liquide 36, une canalisation 31 reliant le réservoir 33 à la tête de nébulisation 30 et une canalisation 34 équipée d'une pompe ou une vanne électrique 35, reliant le réservoir 1 au réservoir 33. Les réservoirs 1 et 33 sont soumis à la pression atmosphérique Patm. La tête de nébulisation 30, sensiblement horizontale, comprend un tube capillaire 30-1 et une buse 30-2 d'éjection du liquide. La tête de nébulisation revêt généralement la forme d'une aiguille creuse d'un diamètre intérieur inférieur au millimètre et d'une longueur de quelques centimètres, dont le corps forme le tube capillaire 30-1 et dont l'extrémité distale, biseautée, forme la buse d'éjection 30-2. La tête de nébulisation 30 est couplée mécaniquement à un moyen vibrant, généralement un transducteur piézo-électrique TPE à résonateur. Le transducteur TPE est excité par un signal alternatif Sv fourni par un circuit EXCT. Le circuit EXCT est piloté par un circuit de contrôle CNTCT qui définit la durée de cycles de nébulisation durant lesquels la buse 30-2 émet du liquide nébulisé, et la durée des périodes de repos entre les cycles de nébulisation. Le circuit CNTCT contrôle également la pompe 35 et ajuste le niveau de liquide dans le réservoir intermédiaire 33, qu'il surveille au moyen d'un détecteur de niveau 33a.

Lorsque le signal d'excitation Sv est appliqué au transducteur TPE, la tête de nébulisation entre en résonance et des gouttelettes 32 de liquide 36 sont éjectées, formant une sorte de brouillard de gouttelettes ou "jet de nébulisation". Pendant la nébulisation, la tête de nébulisation 30 est alimentée en liquide par capillarité et par gravité, la pression à l'entrée du tube capillaire 30-1 étant fonction de la hauteur h1 de la colonne de liquide entre le réservoir intermédiaire 33 et le tube capillaire 30-1.

La figure 2 représente un mode de réalisation d'un appareil de nébulisation. Sur la figure 2, l'appareil de nébulisation DF1 comprend un réservoir principal 1 constitué par exemple d'une cartouche amovible, un circuit de nébulisation NBCT tel que celui représenté sur la figure 1, alimenté en liquide à nébuliser par la cartouche 1, un transducteur piézo-électrique TPE à résonateur couplé mécaniquement à une tête de nébulisation du circuit NBCT, un circuit d'excitation EXCT fournissant un signal alternatif d'excitation Sv au transducteur TPE, et un circuit de contrôle comprenant un microprocesseur µP commandant le circuit de nébulisation NBCT et le circuit d'excitation EXCT. Le microprocesseur µP est alimenté par un circuit d'alimentation ALT et est connecté à un contact PC de présence de la cartouche 1, et à un sélecteur de quantité moyenne de liquide à nébuliser par unité de temps SPO. Dans le cas d'un parfum, cette quantité est équivalente à une puissance olfactive. Le sélecteur SPO permet par exemple de sélectionner une valeur de durée de période de repos parmi trois valeurs telles que 30 s, 60 s et 120 s. Si le temps du cycle de nébulisation est de 300 ms et la concentration en produit actif du liquide à nébuliser est de 5 ou 6%, l'appareil peut traiter des volumes de l'ordre de 300 m3, 150 m3 et 75 m3, avec respectivement 30 s, 60 s et 120 s de période de repos.

Selon un mode de réalisation, l'appareil de nébulisation comprend un sélecteur de concentration SC connecté au microprocesseur µP et permettant d'indiquer à ce dernier la concentration en produit actif, par exemple en parfum, du liquide contenu dans la cartouche. Le sélecteur de concentration SC comporte par exemple trois ou quatre positions pour sélectionner la concentration du produit actif contenu dans la cartouche 1. L'information de concentration figure par exemple sur la cartouche. Le sélecteur de quantité moyenne de liquide à nébuliser par unité de temps SPO peut comporter également trois positions pour effectuer une sélection parmi trois puissances olfactives. Le microprocesseur µP est programmé pour adapter automatiquement la durée des cycles de nébulisation du liquide contenu dans la cartouche et la durée des périodes de repos entre les cycles de nébulisation en fonction des positions indiquées par les deux sélecteurs SC et SPO. Si le sélecteur SC comporte quatre positions et le sélecteur SPO trois positions, le microprocesseur µP peut ainsi adapter la nébulisation à douze volumes à traiter différents.

La concentration en produit actif du liquide à nébuliser contenu dans la cartouche 1 peut varier entre 2,5% et 10%. En effet, pour satisfaire aux exigences de certaines normes et à des contraintes de coût, la concentration ne peut guère excéder 10 %, soit le double ou le triple de la concentration en parfum des liquides actuellement commercialisés pour être diffusés. Cependant, si on accepte pour certaines applications ou certains produits à diffuser de se référer à d'autres normes, la concentration pourra être augmentée, par exemple jusqu'à 60%. La concentration en produit actif ne peut également guère être inférieure à 2,5%, pour garantir que le seuil de perception olfactive soit dépassé avec des durées moyennes de cycles de nébulisation et de périodes de repos entre cycles.

Bien entendu, chaque cycle de nébulisation peut comprendre des microcycles de nébulisation espacés par des périodes de repos de courte durée de l'ordre de celle des microcycles de nébulisation.

Le tableau suivant donne à titre d'exemple des volumes traités et des durées de cartouche pour différentes valeurs de concentration en produit actif du liquide nébulisé, et de durées des cycles de nébulisation et des périodes de repos :

**Tableau 1**

| Concentration (%) | Durée cycle nébul. (ms) | Durée périodes de repos (s) | Quantité liquide nébulisé (mg/jour) | Volume traité (m3) | Durée cartouche (jour) |
|---|---|---|---|---|---|
| 3 | 250 | 300 | 775,19 | 13,95 | 399,90 |
| 6 | 100 | 120 | 775,19 | 27,91 | 399,90 |
| 10 | 300 | 120 | 2325,58 | 139,53 | 133,3 |
| 15 | 300 | 120 | 2325,58 | 209,30 | 133,3 |
| 15 | 300 | 60 | 4651,16 | 418,60 | 66,65 |
| 10 | 100 | 240 | 387,60 | 23,26 | 799,80 |

Les valeurs du tableau 1 ont été obtenues pour un débit de liquide nébulisé de 0,0108 mg/ms, une quantité de produit actif nébulisé par unité de volume de 1,67 mg/m3 et une quantité de liquide dans la cartouche de 310 g. Un volume minimum de moins de 15 m3 peut ainsi être traité avec une autonomie de cartouche supérieure à un an. Par ailleurs, un volume supérieur à 400 m3 peut également être traité avec le même appareil, avec une autonomie de cartouche supérieure à 2 mois. Le dernier exemple donné dans le tableau 1 montre qu'il peut être choisi de réduire la stabilité olfactive en augmentant sensiblement la durée des périodes de repos, pour privilégier la limitation de la consommation électrique de l'appareil ou la limitation de la consommation de l'appareil en liquide à nébuliser.

La possibilité d'agir sur un paramètre supplémentaire, à savoir la concentration du liquide à nébuliser, offre une souplesse de configuration de l'appareil supplémentaire, permettant notamment, dans le cas où l'appareil est alimenté par des piles électriques, d'ajuster la consommation électrique de l'appareil afin que la durée des piles électriques soit un multiple entier au moins égal à 1 de la durée de la cartouche. Cette disposition permet de limiter les opérations de maintenance.

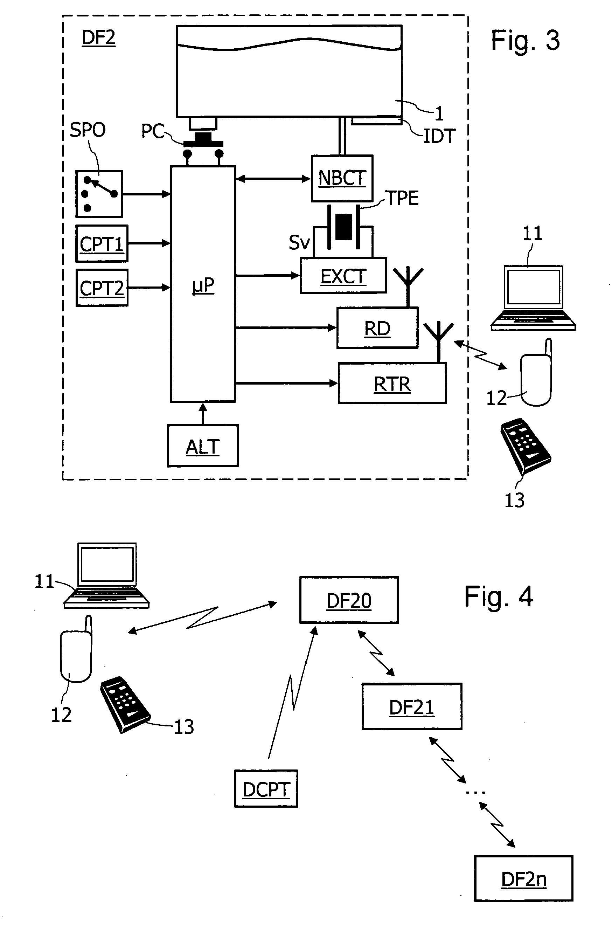

La figure 3 représente un autre mode de réalisation d'un appareil de nébulisation. Sur la figure 3, l'appareil de nébulisation DF2 diffère de l'appareil DF1 en ce qu'il ne comporte pas de sélecteur de concentration SC, mais un dispositif de lecture RD connecté au microprocesseur µP, pour lire une étiquette électronique IDT, par exemple de type RFID, fixée sur la cartouche 1. L'information de concentration du liquide dans la cartouche 1 est mémorisée dans l'étiquette IDT et lue par le dispositif RD qui fournit cette information au microprocesseur µP.

L'appareil DF2 peut comprendre également un ou plusieurs capteurs CPT1, CPT2 connectés au microprocesseur µP. Les capteurs CPT1, CPT2 comprennent par exemple un capteur de présence CPT1 pour détecter la présence d'un ou plusieurs usagers.

Le microprocesseur µP peut ainsi être programmé pour diffuser un parfum dans des toilettes individuelles. A cet effet, le microprocesseur est programmé pour exécuter une phase d'attente durant laquelle il réalise périodiquement un cycle de nébulisation de parfum, par exemple toutes les 3 minutes, afin de maintenir une odeur d'accueil. A la suite de la détection de la présence d'un usager, le microprocesseur passe dans une phase active durant laquelle il réalise un cycle de nébulisation toutes les 5 à 10 secondes. A la suite de la détection du départ de l'usager, le microprocesseur reste dans la phase active pendant un certain temps, par exemple 2 minutes, avant de retourner en phase d'attente.

Pour un usage de l'appareil dans des lieux publics comme des toilettes publiques, le microprocesseur peut être programmé pour adapter lui-même la durée des cycles de nébulisation et la durée des périodes de repos entre les cycles de nébulisation, en fonction de la fréquentation du lieu. Il est en effet peu économique de diffuser la même quantité de produit bactériostatique dans un lieu quelle que soit la fréquentation du lieu. Le capteur CPT1 connecté au microprocesseur peut alors fournir une information relative à la fréquentation du lieu où est installé l'appareil DF2, par exemple un nombre de passages de personnes.

Dans un mode de réalisation, les capteurs CPT1, CPT2 comprennent un capteur de température CPT2. Il s'avère en effet que le débit de liquide nébulisé varie en fonction de la température ambiante. Le microprocesseur peut ainsi être programmé pour maintenir sensiblement constante la quantité de liquide nébulisée à chaque cycle de nébulisation en tenant compte de la température ambiante fournie par le capteur CPT2.

Dans un mode de réalisation, l'appareil comprend un dispositif de transmission sans fil RTR permettant de configurer à distance le fonctionnement de l'appareil DF2 à partir d'un dispositif de commande déporté (télécommande dédiée 13, ordinateur 11 ou téléphone mobile 12 de type "Smartphone"). Le dispositif de transmission RTR comprend par exemple une interface de communication sans fil de type Bluetooth, ou WiFi, ou bien une interface de communication filaire par exemple de type Ethernet, USB ou par courant porteur. Le dispositif RTR peut en outre être configuré pour émettre par exemple vers l'ordinateur 11 des informations relatives à l'état de l'appareil DF2.

Dans un mode de réalisation simplifié, la configuration à distance est réduite au réglage du paramètre de puissance olfactive qui était introduit par le sélecteur SPO dans le mode de réalisation de la figure 2.

Dans un mode de réalisation plus évolué, la configuration à distance permet à l'utilisateur équipé du dispositif de commande déporté 11, 12 ou 13 d'introduire des informations telles que les dimensions ou le volume de la pièce où est installé l'appareil DF2, la fonction de la pièce (hall d'accueil, salle de réunion, toilettes, séjour, ...), la position de l'appareil par rapport à des ouvertures ou des bouches d'aération, et un mode de diffusion du liquide à nébuliser comme par exemple "pic d'odeur" et "odeur d'accueil". Une application installée dans le dispositif de commande déporté 11, 12, 13 détermine en fonction des informations introduites par l'utilisateur et de la concentration du liquide dans la cartouche, des durées de cycles de nébulisation et des durées de période de repos et transmet ces informations à l'appareil DF2. Les durées calculées peuvent être déterminées pour des valeurs ou des plages des valeurs fournies par les capteurs CPT1, CPT2. Alternativement, l'application peut déterminer une quantité de liquide à nébuliser à chaque cycle de nébulisation, la durée du cycle de nébulisation étant ajustée par le microprocesseur µP en fonction de la température fournie par le capteur CPT2 et la quantité de liquide à nébuliser reçue de l'application. Si le volume à traiter est supérieur à la capacité de traitement d'un appareil ou si la quantité de liquide à nébuliser doit être plus importante (par exemple supérieure au seuil de dépôt de gouttes au voisinage de l'appareil), plusieurs appareils peuvent être installés dans le même volume. Dans ce cas, l'application détermine les paramètres de nébulisation (durée des périodes de repos et des cycles de nébulisation ou quantité de liquide à nébuliser à chaque cycle) de chaque appareil installé dans le volume en tenant compte de la présence des autres appareils.

Bien entendu, tout ou partie des traitements effectués par l'application installée dans le dispositif de commande déporté 11, 12, 13 peuvent être réalisés par le microprocesseur µP. Egalement, le paramètre de concentration du liquide contenu dans la cartouche 1 peut être fourni par l'utilisateur par l'intermédiaire du dispositif de commande 11, 12, 13. Dans ce cas, il n'est pas nécessaire que l'appareil soit équipé du dispositif de lecture RD et que la cartouche 1 comporte une étiquette électronique.

Dans un mode de réalisation illustré par la figure 4, plusieurs appareils de nébulisation DF20, DF21, ..., DF2n du type DF2 communiquent entre eux par l'intermédiaire du dispositif de transmission RTR équipant chacun des appareils. L'un de ces appareils, par exemple DF20 est considéré comme l'appareil maître et retransmet des ordres provenant d'un dispositif de commande à distance (ordinateur 11, téléphone mobile 12, télécommande dédiée 13) destinés aux autres appareils DF21, ..., DF2n. D'une manière générale, chaque appareil retransmet les ordres qui ne lui sont pas destinés vers les autres appareils. Inversement, chaque appareil retransmet vers le dispositif de commande les informations d'état de fonctionnement reçues des autres appareils. A cet effet, chaque message transmis par l'un des appareils ou le dispositif de commande à distance comporte un identifiant permettant à chacun des appareils ou au dispositif de commande de déterminer si le message lui est destiné ou non.

Ces dispositions permettent notamment de minimiser la puissance d'émission nécessaire dans le cas de transmissions sans fil, et donc la consommation électrique des appareils. Ces dispositions permettent également de minimiser les temps de programmation et de supervision des appareils lorsqu'ils sont nombreux à être installés dans un même espace ou un même bâtiment comme par exemple une tour de bureaux.

Le dispositif de commande à distance 11, 12, 13 peut également recevoir des informations de capteurs déportés DCPT, par exemple un détecteur ou un compteur de personnes. Ainsi, comme chacun des appareils DF20-DF2n se comporte comme un relais de transmission, il n'est pas nécessaire que le dispositif de transmission RTR équipant chacun des appareils présente une grande portée.

Il apparaîtra clairement à l'homme de l'art que la présente invention est susceptible de diverses variantes de réalisation et applications. En particulier, l'invention n'est pas limitée à un ajustement de la durée des cycles de nébulisation et de la durée des périodes de repos entre les cycles de nébulisation, simultanément. La concentration en produit actif du liquide à nébuliser n'est limitée à 15% qu'en raison de normes et de contraintes d'étiquetage et de coût. Par conséquent, aucun obstacle technique ne s'oppose à nébuliser un liquide contenant plus de 15% de produit actif.

## Revendications

1. Procédé de commande d'un appareil de nébulisation de liquides dans l'air (DF1, DF2, DF20-DF2n), le procédé comprenant des étapes de :
commande de l'appareil pour nébuliser un liquide dans l'air conformément à des cycles de nébulisation durant lesquels un liquide est nébulisé dans l'air, les cycles de nébulisation étant espacés par des périodes de repos, et
ajustement de la durée des périodes de repos en fonction d'une quantité moyenne de liquide à nébuliser par unité de temps sélectionnée,
**caractérisé en ce qu'**il comprend une étape d'ajustement de la durée des périodes de repos en fonction d'un paramètre de concentration en produit actif du liquide à nébuliser.

2. Procédé selon la revendication 1, comprenant une étape d'ajustement de la durée des cycles de nébulisation ou de la quantité de liquide à nébuliser à chaque cycle de nébulisation en fonction de la quantité de moyenne de liquide à nébuliser par unité de temps sélectionnée et du paramètre de concentration en produit actif contenu dans le liquide à nébuliser.

3. Procédé selon la revendication 1 ou 2, dans lequel la durée de chaque cycle de nébulisation est ajustée à une valeur comprise entre une centaine de millisecondes et quelques secondes, et la durée de chaque période de repos entre les cycles de nébulisation est ajustée à une valeur comprise entre plusieurs dizaines de secondes et quelques minutes.

4. Procédé selon l'une des revendications 1 à 3, dans lequel l'ajustement de la durée des périodes de repos en fonction de la concentration en produit actif du liquide contenu dans la cartouche peut être effectué lorsque la concentration présente une valeur comprise entre 2,5% et 15%, et de préférence entre 2,5% et 60%.

5. Procédé selon l'une des revendications 1 à 4, comprenant une étape d'ajustement de la durée des périodes de repos et/ou des cycles de nébulisation en fonction d'une information fournie par un capteur (CPT1, CPT2), tel qu'un capteur de la température ambiante.

6. Procédé selon l'une des revendications 1 à 5, dans lequel les durées respectives de périodes de repos successives sont ajustées de manière à produire des "pics d'odeurs", en tenant compte de caractéristiques neurosensorielles olfactives humaines et en prévoyant un temps supplémentaire d'attente.

7. Procédé selon l'une des revendications 1 à 6, dans lequel la concentration en produit actif du liquide à nébuliser est sélectionnée en fonction du volume à traiter par l'appareil et afin que la durée de fonctionnement de piles électriques d'alimentation de l'appareil soit un multiple entier au moins égal à 1 de la durée d'une cartouche amovible (1) alimentant l'appareil en liquide à nébuliser.

8. Procédé selon l'une des revendications 1 à 7, comprenant une étape de configuration du mode de fonctionnement de l'appareil consistant à déterminer la durée des périodes de repos et des cycles de nébulisation ou la quantité de liquide à nébuliser à chaque cycle de nébulisation en fonction d'au moins un paramètre appartenant à l'ensemble comprenant des dimensions et une fonction d'une pièce où est installé l'appareil, un nombre d'appareils installés dans la pièce, la position de l'appareil dans la pièce par rapport à des ouvertures et des bouches d'aération, un mode de diffusion du liquide à nébuliser, la consommation électrique de l'appareil, et la consommation de l'appareil en liquide à nébuliser.

9. Procédé selon l'une des revendications 1 à 8, comprenant la transmission de paramètres de fonctionnement à une pluralité d'appareils de nébulisation de liquides dans l'air.

10. Appareil de nébulisation de liquides dans l'air (DF1, DF2, DF20-DF2n), comprenant :
un circuit de nébulisation (NBCT),
une cartouche amovible (1) contenant un liquide à nébuliser relié au circuit de nébulisation, et
une unité de commande (µP) du circuit de nébulisation configurée pour nébuliser le liquide dans l'air conformément à des cycles de nébulisation durant lesquels un liquide est nébulisé dans l'air, les cycles de nébulisation étant espacés par des périodes de repos, l'unité de commande étant configurée pour ajuster la durée des périodes de repos en fonction d'une quantité moyenne de liquide à nébuliser par unité de temps sélectionnée,
**caractérisé en ce que** l'unité de commande (µP) est configurée pour ajuster la durée des périodes de repos en fonction d'un paramètre de concentration en produit actif du liquide à nébuliser.

11. Appareil selon la revendication 10, dans lequel l'unité de commande (µP) est configurée pour ajuster la durée des cycles de nébulisation en fonction de la quantité moyenne de liquide à nébuliser sélectionnée et du paramètre de concentration en produit actif contenu dans le liquide à nébuliser.

12. Appareil selon la revendication 10 ou 11, dans lequel l'unité de commande (µP) est configurée pour ajuster la durée de chaque cycle de nébulisation à une valeur comprise entre une centaine de millisecondes et quelques secondes, et ajuster la durée de chaque période de repos entre les cycles de nébulisation à une valeur comprise entre plusieurs dizaines de secondes et quelques minutes.

13. Appareil selon l'une des revendications 10 à 12, dans lequel l'unité de commande (µP) est configurée pour pouvoir ajuster la durée des périodes de repos en fonction de la concentration en produit actif du liquide contenu dans la cartouche, lorsque la concentration en produit actif présente une valeur comprise entre 2,5% et 15%, et de préférence entre 2,5% et 60%.

14. Appareil selon l'une des revendications 10 à 13, dans lequel l'unité de commande (µP) est configurée pour ajuster la durée des périodes de repos et/ou des cycles de nébulisation en fonction d'une information fournie par un capteur (CPT1, CPT2) relié à l'unité de commande, tel qu'un capteur de la température ambiante.

15. Appareil selon l'une des revendications 10 à 14, dans lequel l'unité de commande (µP) est configurée pour déterminer les durées respectives de périodes de repos successives de manière à produire des "pics d'odeurs", en tenant compte de caractéristiques neurosensorielles olfactives humaines et en prévoyant un temps supplémentaire d'attente.

16. Appareil selon l'une des revendications 10 à 15, comprenant un dispositif de transmission (RTR) connecté à l'unité de commande (µP), pour recevoir d'un dispositif de commande à distance (11, 12, 13) des paramètres de fonctionnement, l'unité de commande étant configurée pour déterminer la durée des périodes de repos et/ou la durée des cycles de nébulisation en fonction des paramètres reçus, les paramètres reçus du dispositif de commande pouvant comprendre des dimensions et une fonction d'une pièce où est installé l'appareil (DF2, DF20-DF2n), ou un mode de diffusion du liquide à nébuliser.

17. Appareil selon la revendication 16, dans lequel l'unité de commande (µP) est configurée pour transmettre vers le dispositif de commande à distance (11, 12, 13) des informations d'état de l'appareil, le dispositif de transmission (RTR) étant relié au dispositif de commande à distance par l'intermédiaire d'une liaison sans fil ou filaire.

18. Appareil selon l'une des revendications 16 et 17, configuré pour retransmettre un message provenant du dispositif de commande à distance (11, 12, 13) et destiné à un autre appareil, et retransmettre un message reçu d'un autre appareil et destiné au dispositif de commande à distance.

19. Système de nébulisation de liquides dans l'air, comprenant plusieurs appareils de nébulisation de liquides dans l'air (DF20-DF2n),
**caractérisé en ce que** les appareils sont conformes à l'appareil selon l'une des revendications 16 à 18.
